# NOUVEAU FASCICULE DE BREVET EUROPEEN

(11) **EP 0 661 076 B2**
(45) Date de publication et mention de la décision concernant l'opposition: **28.04.2004**
(45) Mention de la délivrance du brevet: 01.12.1999
(21) Numéro de dépôt: 94403057.6
(22) Date de dépôt: 30.12.1994
(51) Int. Cl.: A61N 1/368

(54) **Dispositif de commande d'un stimulateur cardiaque asservi à au moins un paramètre physiologique**
Vorrichtung zum Steuern eines Herzschrittmachers als Funktion von mindestens einem physiologischen Parameter
Device for controlling a pacemaker by monitoring at least one physiological parameter

(30) Priorité: 31.12.1993 FR 9315939
(43) Date de publication de la demande: 05.07.1995
(73) Titulaire: ELA MEDICAL (Société anonyme), 92541 Montrouge (FR)
(72) Inventeur: Bonnet, Jean-Luc, F-92170 Vanves (FR); Bonhour, Anne, F-75013 Paris (FR)
(74) Mandataire: Dupuis-Latour, Dominique

(56) Documents cités:
- EP-A- 0 360 668
- EP-A- 0 448 193
- EP-A- 0 488 841
- EP-A- 0 559 193
- FR-A- 2 544 989
- US-A- 5 074 304
- US-A- 5 085 215

## Description

L'invention concerne le domaine des dispositifs implantables actifs, et plus particulièrement celui des stimulateurs cardiaques dont la fréquence de stimulation est asservie à un paramètre physiologique.

Les stimulateurs cardiaques de la famille dite "double chambre" ont pour fonction, entre autres, de suivre le rythme spontané détecté au niveau de l'oreillette et de stimuler en synchronisme le ventricule. Toutefois, cette synchronisation présente une limite supérieure appelée "fréquence maximale de stimulation ventriculaire" . En effet, si le patient développe une crise de tachycardie auriculaire (également appelée "tachycardie supra-ventriculaire" ou TSV), il est nécessaire de limiter la synchronisation à une plage donnée, afin d'éviter une stimulation trop rapide du ventricule au-delà d'une fréquence maximale supportable par l'individu.

Plusieurs solutions ont été mises en oeuvre pour commander la stimulation ventriculaire dans le cas d'une telle accélération excessive de la fréquence auriculaire. Tous ces dispositifs ont pour point commun d'essayer de garder une certaine synchronisation au-delà de la fréquence maximale et, si la fréquence auriculaire continue à dépasser la fréquence maximale de synchronisation, ils déclenchent un mode de stimulation asynchrone, tendant vers une fréquence ventriculaire plus basse. Dès que la fréquence auriculaire repasse au-dessous de la limite maximale de synchronisation, une phase de resynchronisation progressive est entamée.

Le FR-A-2 544 989 décrit un procédé de synchronisation au-delà de la fréquence maximale de synchronisation par allongement du délai auriculo-ventriculaire (délai AV) pour une première plage de fréquences auriculaires, puis la non-écoute d'un signal auriculaire parmi quelques-uns, dans une plage de fréquences supérieures. Si ces conditions persistent, la stimulation ventriculaire se désynchronise de la fréquence auriculaire et diminue jusqu'à une fréquence de base. Ce mode de fonctionnement est appelé "repli" (*fallback*).

Le US-A-4 932 046 reprend, pour l'essentiel, la même idée. Toutefois, la désynchronisation y est immédiate dès le franchissement par l'oreillette de la fréquence maximale de synchronisation, et la fréquence ventriculaire tend alors vers la fréquence de consigne indiquée par un capteur physiologique.

Le EP-A-0 448 193 présente une approche différente du problème. L'information délivrée par le capteur physiologique sert à déterminer la fréquence maximale de synchronisation du ventricule et, lorsque l'intervalle de couplage détecté par le stimulateur dépasse trop souvent cette fréquence maximale de synchronisation du ventricule, le stimulateur passe en mode de désynchronisation, cette désynchronisation suivant des phases semblables à celles décrites dans les cas précédents.

Dans toutes ces propositions de l'art antérieur, l'information du capteur n'est cependant jamais utilisée directement pour établir et confirmer la survenance d'une accélération pathologique de l'oreillette.

Un premier but de la présente invention est, précisément, d'utiliser directement l'information du capteur pour détecter et confirmer la présence d'une tachycardie supra-ventriculaire afin de déclencher en réponse une phase de désychronisation, suivie si nécessaire d'une phase de repli.

Un autre but de l'invention est de combler les absences de détection en cas de fibrillation auriculaire, de façon à bien confirmer la nécessité d'une désynchronisation et éventuellement d'un repli.

A cet effet, l'invention propose un stimulateur présentant les caractéristiques énoncées dans la revendication 1. Les sous-revendications visent des formes de mise en oeuvre advantageuses.

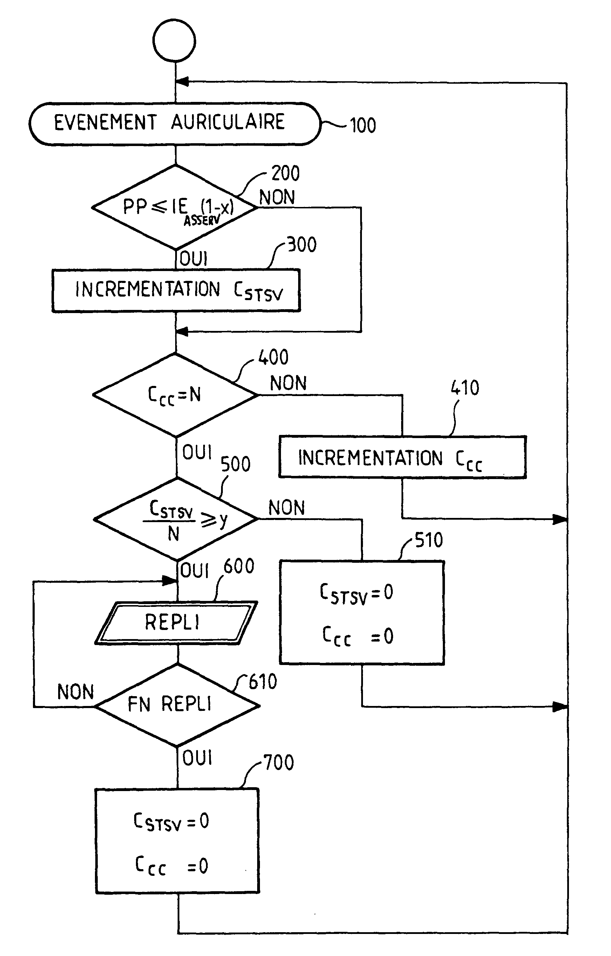

D'autres caractéristiques et avantages de l'invention apparaîtront à la lecture de la description détaillée ci-dessous d'un exemple de mise en oeuvre, en référence à la figure unique annexée, qui est un organigramme illustrant le déroulement du procédé avantageusement utilisé dans l'invention.

Tout d'abord, on notera que l'algorithme illustré peuvent être avantageusement mis en oeuvre par programmation d'un microprocesseur du dispositif implantable, ou à l'aide de circuits dédiés équivalents ; une telle réalisation est à la portée du spécialiste de cette technique et ne sera pas décrite plus en détail pour cette raison.

Par ailleurs, cet algorithme n'est mis en oeuvre que lorsque le capteur physiologique (ou au moins l'un des capteurs physiologiques) est en activité et que la fonction de repli a été préalablement activée par programmation.

On surveille alors le rythme auriculaire, qu'il soit spontané ou stimulé. L'intervalle de temps (cycle cardiaque) entre deux événements auriculaires consécutifs, spontanés ou stimulés, sera appelé "intervalle de couplage" ou "intervalle PP". L'acquisition de cette donnée est effectuée à chaque battement auriculaire, et la donnée correspondante est mise à jour de façon concomitante dans une mémoire ou un compteur du dispositif implanté.

Le dispositif comporte par ailleurs un circuit d'asservissement opérant en réponse à un capteur physiologique (ou à divers capteurs physiologiques). Ce circuit calcule et délivre un signal de consigne que l'on appellera "intervalle d'échappement d'asservissement" et que l'on notera IE_{ASSERV}, qui servira à déterminer la fréquence de stimulation en mode asservi.

Le dispositif comporte en outre un premier compteur indiquant le nombre total de cycles cardiaques analysés (noté ci-dessous C_{CC}).

Il est également prévu un second compteur qui compte un nombre de cycles (noté ci-dessous C_{STSV}) que l'on appellera "cycles en suspicion de TSV" , définis comme étant des cycles pour lesquels l'intervalle de couplage PP est plus court qu'une valeur égale à l'intervalle d'échappement d'asservissement (valeur de consigne) diminué d'une quantité ou proportion donnée que l'on appellera "facteur de prématurité". Dans l'exemple décrit, on définit ce "facteur de prématurité" comme étant un pourcentage *x* donné de l'intervalle d'échappement d'asservissement, mais ce choix n'est pas limitatif, et l'on pourrait également exprimer le facteur de prématurité sous forme absolue, par une durée programmable.

Essentiellement, le procédé consiste à examiner une séquence de N cycles cardiaques et, si sur ces N cycles le nombre de cycles en suspicion de TSV atteint un pourcentage programmable du nombre total de cycles de la séquence, considérer qu'il y a présence effective d'une TSV et engager de repli.

L'algorithme correspondant est illustré sur la figure unique.

Cet algorithme débute à l'étape 100, lorsque l'on détecte un événement auriculaire (qu'il soit spontané ou stimulé).

À l'étape 200, on compare l'intervalle de couplage PP de cet événement à l'intervalle d'échappement d'asservissement IE_{ASSERV} déterminé par le capteur, diminué du facteur de prématurité *x*.

Si l'intervalle de couplage PP est inférieur à ce terme, ce qui signifie que la fréquence de l'oreillette est nettement supérieure à la fréquence de consigne délivrée par le capteur, on incrémente (étape 300) le compteur C_{STSV} de cycles en suspicion de TSV. Dans le cas contraire, on passe directement à l'étape 400.

À cette étape 400, on détermine si le compte de cycles cardiaques C_{CC} a atteint une limite N prédéterminée, correspondant à l'étendue de la séquence d'analyse. Si cette limite n'est pas atteinte, le compteur C_{CC} est incrémenté (étape 410), et le dispositif se place en attente du prochain événement auriculaire, en 100. Dans le cas contraire, on examine, à l'étape 500, la proportion du nombre de cycles en suspicion de TSV par rapport au nombre total de cycles N.

Si ce rapport est inférieur à une valeur y programmable, on considère qu'il n'y a pas de TSV avérée et l'on réinitialise (étape 510) les deux compteurs C_{STSV} et C_{CC} puis l'on se met en attente d'un nouvel événement auriculaire, en 100.

En revanche, si le rapport est supérieur ou égal à *y*, c'est-à-dire si l'on a trouvé un grand nombre de cycles suspects parmi les N cycles, on considère qu'il y a eu survenance effective d'une TSV et l'on déclenche en conséquence le mode de repli (étape 600). Ce mode de repli est en lui même connu et on ne le décrira pas en détail ; on pourra par exemple se référer aux publications citées au début de la présente description.

Lorsque le repli prend fin (test de l'étape 610), on réinitialise à l'étape 700 les compteurs C_{STSV} et C_{CC} et l'on se met en attente d'un nouvel événement auriculaire.

Comme on peut le voir, l'information d'intervalle d'échappement de consigne IE_{ASSERV}, qui est dérivée d'un ou plusieurs paramètres physiologiques mesurés par le ou les capteur(s), sert à déterminer s'il y a ou non lieu de déclencher le mode de repli. Elle est donc, de façon caractéristique de l'invention, exploitée avant tout repli. Bien évidemment elle peut également servir, de manière en elle-même connue, à contrôler le déroulement du repli subséquent par action sur le système d'asservissement opérant sous contrôle du ou des capteur(s).

On notera que, bien que l'exemple ci-dessus fasse appel à des comparaisons utilisant des valeurs en pourcentage (telles que *x* et *y*), on pourrait sans sortir du cadre de l'invention utiliser des valeurs absolues (durée ou nombre de cycles, respectivement) en lieu et place des valeurs de pourcentage. De même, certaines valeurs programmables pourraient être déterminées et fixées à l'avance.

Le tableau ci-dessous donne des exemples de valeurs programmables envisageables pour la mise en oeuvre de l'invention.

| | étendue | nominal |
|---|---|---|
| *x* (facteur de prématurité) | 0 à 50% | 37,5 % |
| N (nombre de cycles analysés) | 10 à 256 | 256 |
| *y* (seuil de suspicion de TSV) | 50 à 75 % | 75 % |

## Revendications

1. Un stimulateur cardiaque comprenant un asservissement à au moins un paramètre physiologique et possédant un mode de désynchronisation de la stimulation ventriculaire lorsque le rythme auriculaire est trop rapide,
comprenant des moyens pour déclencher le mode de désynchronisation (REPLI) en réponse à une comparaison de l'intervalle de couplage détecté (PP) avec l'intervalle d'échappement de consigne (IE_{ASSERV}) calculé par l'asservissement,
et des moyens de détection du nombre (C_{STSV}) de cycles en suspicion de tachycardie supra-ventriculaire, aptes à commander conditionnellement lesdits moyens pour déclencher le mode de désynchronisation lorsque la valeur de compte correspondante (C_{STSV}) dépasse un seuil prédéterminé.

2. Le stimulateur de la revendication 1, dans lequel les termes de ladite comparaison comprennent, d'une part, l'intervalle de couplage détecté et, d'autre part, l'intervalle d'échappement de consigne diminué d'un facteur de prématurité.

3. Le stimulateur de la revendication 2, dans lequel le facteur de prématurité est exprimé sous forme d'un pourcentage programmable (*x*) de l'intervalle d'échappement de consigne.

4. Le stimulateur de la revendication 3, dans lequel le pourcentage programmable de l'intervalle d'échappement de consigne est compris entre 0 et 50 %, de préférence environ 37,5 %.

5. Le stimulateur de la revendication 2, dans lequel le facteur de prématurité est exprimé sous forme d'une durée programmable.

6. Le stimulateur de la revendication 5, dans lequel la durée programmable est comprise entre 50 et 300 ms, de préférence environ 150 ms.

7. Le stimulateur de la revendication 1, dans lequel les moyens de détection sont des moyens pour, après avoir comparé l'intervalle de couplage détecté avec l'intervalle d'échappement de consigne, compter le nombre de cycles cardiaques pour lesquels l'intervalle de couplage détecté est inférieur à cet intervalle d'échappement de consigne.

8. Le stimulateur de la revendication 7, dans lequel le seuil donné est exprimé sous forme d'un pourcentage programmable (*y*) du nombre total (N) de cycles cardiaques analysés.

9. Le stimulateur de la revendication 8, dans lequel le pourcentage programmable du nombre total de cycles cardiaques analysés est compris entre 50 et 75 %, de préférence environ 75 %.

10. Le stimulateur de la revendication 7, dans lequel le seuil donné est exprimé sous forme d'un nombre programmable de cycles.

11. Le stimulateur de la revendication 10, dans lequel le nombre programmable de cycles est compris entre 10 et 255, de préférence environ 100.

## Patentansprüche

1. Herzschrittmacher, der eine Regelung mit mindestens einem physiologischen Parameter ausweist, und einen Desynchronisationsmodus der ventrikulären Stimulation besitzt, wenn der Vorhofrythmus zu schnell ist,
umfassend: Mittel zum Auslösen des Desynchronisationsmodus (REPLI) in Antwort auf einen Vergleich des erfassten Kopplungsintervalls (PP) mit dem eingestellten Ausstoßintervall (IE_{ASSERV}), welches durch die Regelung berechnet wird,
und Mittel zur Erfassung der Anzahl (C_{STSV}) der Zyklen bei Verdacht einer supraventrikulären Tachykardie, die geeignet sind, konditional die Mittel zum Auslösen des Desynchronisationsmodus zu steuern, wenn der entsprechende Zählwert (C_{STSV}) eine vorbestimmte Schwelle überschreitet.

2. Herzschrittmacher nach Anspruch 1, in welchem die Bedindungen des Vergleichs einerseits das erfasste Kopplungsintervall und andererseits das eingestellte Ausstoßintervall, vermindert um einen Prämaturitätsfaktor umfassen.

3. Herzschrittmacher nach Anspruch 2, in welchem der Prämaturitätsfaktor in Form eines programmierbaren Prozentsatzes (x) des festgelegten Ausstoßintervalls ausgedrückt ist.

4. Herzschnittmacher nach Anspruch 3, in welchem der programmierbare Prozentsatz des festgelegten Ausstoßintervalls zwischen 0 und 50%, vorzugsweise um 37,5% liegt.

5. Herzschrittmacher nach Anspruch 2, bei dem der Prämaturitätsfaktor in Form einer programmierbaren Dauer ausgedrückt ist.

6. Herzschrittmacher nach Anspruch 5, bei dem die programmierbare Dauer zwischen 50 und 300 ms, vorzugsweise ungefähr 150 ms beträgt.

7. Herzschrittmacher nach Anspruch 1, bei dem die Erfassungsmittel Mittel sind, um, nachdem das erfasste Kopplungsintervall mit dem festgelegten Ausstoßintervall verglichen worden ist, die Anzahl von Herzzyklen zu zählen, bei denen das erfasste Kopplungsintervall kleiner als dieses festgelegte Ausstoßintervall ist.

8. Herzschrittmacher nach Anspruch 7, bei dem der gegebene Schwellwert in Form eines programmierbaren Prozentsatzes (y) der Gesamtanzahl (N) von analysierten Herzzyklen ausgedrückt ist.

9. Herzschrittmacher nach Anspruch 8, bei dem der programmierbare Prozentteil der Gesamtanzahl von analysierten Herzzyklen zwischen 50 und 75%, vorzugsweise um 75% liegt.

10. Herzschrittmacher nach Anspruch 7, bei dem der gegebene Schwellwert in Form einer programmierbaren Anzahl von Zyklen ausgedrückt ist.

11. Herzschrittmacher nach Anspruch 10, bei dem die programmierbare Anzahl von Zyklen zwischen 10 und 255, vorzugsweise um 100 liegt.

## Claims

1. A cardiac pacemaker having an enslavement to at least one physiological parameter and including a mode for desynchronisation of the ventricutar stimulation when the atrial rhythm is too rapid,
including means to trigger the desynchronisation mode (REPLI) in response to a comparison of the coupling interval detected (PP) with the reference escape interval (IE_{ASSERV}) calculated by the enslavement,
and means for detecting the number (C_{STSV}) of cycles which are suspected from supra-ventricular tachycardia, which means are adapted to conditionally control said means for triggering the desynchronisation mode when the corresponding count value (C_{STSV}) exceeds a predetermined threshold. I

2. The pacemaker of claim 1, in which the terms of said comparison comprise, on the one hand, the coupling interval detected and, on the other hand, the reference escape interval decreased by a prematurity factor.

3. The pacemaker of claim 2, in which the prematurity factor is expressed in the form of a programmable percentage (*x*) of the reference escape interval.

4. The pacemaker of claim 3, in which the programmable percentage of the reference escape interval is comprised between 0 and 50 %, preferably approximately 37.5 %.

5. The pacemaker of claim 2, in which the prematurity factor is expressed in the form of a programmable duration.

6. The pacemaker of claim 5, in which the programmable duration is comprised between 50 and 300 ms, preferably approximately 150 ms.

7. The pacemaker of claim 1, in which the detection means are means for, after having compared the coupling interval detected with the reference escape interval, counting the number of cardiac cycles in which the coupling interval detected is less than said reference escape interval.

8. The pacemaker of claim 7, in which the predetermined threshold is expressed in the form of a programmable percentage (*y*) of the total number (N) of cardiac cycles analysed.

9. The pacemaker of claim 8, in which the programmable percentage of the total number of analysed cardiac cycles is comprised between 50 and 75 %, preferably approximately 75 %.

10. The pacemaker of claim 7, in which the predetermined threshold is expressed in the form of a programmable number of cycles.

11. The pacemaker of claim 10, in which the programmable number of cycles is comprised between 10 and 255, preferably approximately 100.
